# EUROPÄISCHE PATENTANMELDUNG

(11) **EP 2 742 931 A1**
(43) Veröffentlichungstag der Anmeldung: **18.06.2014**
(21) Anmeldenummer: 12197073.5
(22) Anmeldetag: 13.12.2012
(51) Int. Cl.: A61K 9/00, A61K 47/32, A61K 36/48

(54) **Topisches Arzneimittel zur Behandlung von Aphten**

(71) Anmelder: LTS LOHMANN Therapie-Systeme AG, 56626 Andernach (DE)
(72) Erfinder: Keikert, Rosemarie, 56567 Neuwied (DE)
(74) Vertreter: Michalski Hüttermann & Partner Patentanwälte

(57) **Zusammenfassung**

Die Erfindung betrifft topische Arzneimittel zur Behandlung von Aphten, wobei die topischen Arzneimittel in Form einer filmförmigen Darreichungsform vorliegen, die einen Film aus einem in Wasser quellfähigen oder wasserlöslichen Polymer oder Polymergemisch, und mindestens einen Wirkstoff umfassen, der aus der Gruppe ausgewählt ist, die aus Gerbstoffen und ätherischen Ölen besteht.

## Beschreibung

Die vorliegende Erfindung betrifft topische Arzneimittel zur Behandlung von Aphten, insbesondere topische Arzneimittel, die in filmförmiger Form vorliegen.

Unter einer "Aphte" wird eine schmerzhafte, von einem entzündlichen Randsaum begrenzte Schädigung einer Schleimhaut verstanden. Bei Aphten handelt es sich um Ulcera mit weißlichem Fibrinbelag. Kleine Aphten weisen einen Durchmesser von weniger als einen Zentimeter auf und heilen zumeist innerhalb von ein bis zwei Wochen ab. In seltenen Fällen können Aphten aber auch größer als einen Zentimeter sein, bis zu 3 cm im Durchmesser. Diese als Major-Form bezeichneten Aphten können über Wochen oder gar Monate persistieren und heilen nur unter Narbenbildung ab. Aphten treten überwiegend an der Schleimhaut des Zahnfleisches, der Tonsillen und der Zunge auf. Seltener sind Schleimhäute des Genitalbereichs von Aphten betroffen.

Aphten sind schmerzhaft. Die Schmerzhaftigkeit einer Aphte korreliert weniger mit ihrer Größe, sie ist vielmehr von der Stelle abhängig, an der die Aphte auftritt. Insbesondere beim Sprechen, Essen und Schlucken macht sich der Schmerz bemerkbar. Daher beeinträchtigen Aphten das Wohlbefinden eines Betroffenen erheblich. Insofern ist eine Behandlung von Aphten gewünscht, auch wenn sie von selbst abheilen, um schneller wieder schmerzfrei zu sein. Zur symptomatischen Behandlung von Aphten werden schmerzstillende Wirkstoffe wie beispielsweise Lidocain, Polidocanol oder Benzydamin in Form von Sprays, Gurgellösungen, Gelen oder Salben angeboten. Es werden auch adstringierende Mittel wie Rhabarberwurzelextrakt, Myrrhentinktur, Silbernitrat, Phenolsulfonsäureverbindungen, das Kresol-Sulfonsäure-Kondensat Policresulen, Zinksulfat oder Wasserstoffperoxidlösung verabreicht. Als verschreibungspflichtiges Mittel zur Behandlung von Aphten kann das entzündungshemmende Triamcinolonacetonid genannt werden. Alternative entzündungshemmende Mittel, die zur Behandlung von Aphten zur Anwendung kommen, sind beispielsweise australisches Teebaumöl, Melissenextrakt sowie Kamillen- oder Salbeitee.

Die Offenlegungsschrift DE 10 2004 002 001 A1 offenbart die Verwendung von Hyaluronsäure zur Behandlung von entzündlichen Erkrankungen. Neben anderen Verabreichungsformen werden auch Okklusivfolien erwähnt. Diese Hyaluronsäure enthaltenden Okklusivfolien werden auf die Haut aufgebracht und decken die entzündete Stelle ab. Als eine der entzündlichen Erkrankungen, die mit Hyaluronsäure behandelbar sein soll, werden Aphten genannt.

Das Dokument DE 696 13 840 T1 betrifft Tabletten, die einen nicht anästhesierenden Wirkstoffkern mit Langzeitwirkung aufweisen, der mit einem anästhesierenden Überzug versehen ist. Der Kern kann eine hydrophile Schubschicht aufweisen, die ein hydrophiles Polymer wie Carbopol enthalten kann. Neben den Tabletten wird eine wirkstoffhaltige Polymerfolie zur Behandlung von Aphten erwähnt, die Feuchtigkeit aus der Wangenschleimhaut absorbiert und sich an die Membranoberfläche haftet. Allerdings wird als nachteilig beschrieben, dass eine Irritation der Behandlungsstelle bei Verabreichen eines zu Irritationen führenden Wirkstoffs stärker ausfällt, wenn die Verabreichung mittels derartiger Polymerfolien erfolgt. Dieser Effekt ist insbesondere problematisch, wenn die Behandlungsstelle eine schmerzhafte Entzündung ist, weil der Schmerz erhöht wird.

Die Patentschrift DE 694 29 964 T2 offenbart feste Zubereitungen zur Behandlung von Schleimhauterkrankungen. Bei diesen festen Zubereitungen handelt es sich um Presslinge, enthaltend ein Homo- oder Copolymer der Acrylsäure, einen Celluloseether und Gelatine.

Die aus dem Stand der Technik bekannten Medikamente zur Behandlung von Aphten sind nicht in jeder Hinsicht optimal. So haben Salben, Mundwasser, Tinkturen und Pasten den Nachteil, dass sie nur für kurze Zeit auf der Aphte haften. Hafttabletten weisen den Nachteil auf, dass ihre Applikation für den Patienten schmerzhaft ist, denn es handelt sich um harte Presslinge, die Druck auf die Aphte ausüben. Daher besteht ein Bedarf an einem topischen Arzneimittel, das leicht zu applizieren ist, das lange Zeit auf der Aphte verweilt und dessen Anwendung dem Patienten nicht noch mehr Schmerzen bereitet.

Der vorliegenden Erfindung lag daher die Aufgabe zugrunde, ein topisches Arzneimittel zur Behandlung von Aphten bereitzustellen, das einen oder mehrere Wirkstoffe kontinuierlich abgibt, im Mund nicht schmilzt und bei dem gewährleistet ist, dass sich der Wirkstoff oder die Wirkstoffe ausreichend in der Mundschleimhaut verteilen, und bei dessen Anwendung Hautreizungen weitgehend vermieden werden.

Die Aufgabe wird durch ein topisches Arzneimittel in Form einer filmförmigen Darreichungsform gelöst, die ein in Wasser quellfähiges oder wasserlösliches Polymer oder Polymergemisch, und mindestens einen zur Behandlung von Aphten geeigneten Wirkstoff umfasst. Die erfindungsgemäße filmförmige Darreichungsform ist leicht auf einer Aphte zu applizieren, kann dort verweilen und den in ihr enthaltenen Wirkstoff kontinuierlich abgeben, da sie sich erst über einen längeren Zeitraum hinweg, also über einen Zeitraum von mehr als einer Stunde, auflöst. Überraschenderweise bereitet die Applikation der erfindungsgemäßen Darreichungsform dem Patienten keine zusätzlichen Schmerzen.

In einem ersten Aspekt betrifft die Erfindung ein topisches Arzneimittel zur Behandlung von Aphten, das als filmförmige Darreichungsform vorliegt.

In einem zweiten Aspekt betrifft die Erfindung ein Verfahren zur Herstellung der filmförmigen Darreichungsform zur Behandlung von Aphten.

In einem dritten Aspekt betrifft die Erfindung die Verwendung der filmförmigen Darreichungsform zur Behandlung von Aphten.

In einem vierten Aspekt betrifft die Erfindung ein Verfahren zur Behandlung von Aphten mit Hilfe der filmförmigen Darreichungsformen.

Gemäß dem ersten Aspekt der Erfindung liegt das topische Arzneimittel zur Behandlung von Aphten in Form einer filmförmigen Darreichungsform vor. Die filmförmige Darreichungsform umfasst ein in Wasser quellfähiges oder wasserlösliches Polymer oder ein in Wasser quellfähiges oder wasserlösliches Polymergemisch. Ferner umfasst die filmförmige Darreichungsform mindestens einen Wirkstoff zur Behandlung von Aphten, d.h. mindestens einen zur Behandlung von Aphten geeigneten Wirkstoff.

Die erfindungsgemäße filmförmige Darreichungsform besitzt eine solche Eigenklebrigkeit, dass ein dauernder Kontakt mit der Schleimhaut sichergestellt ist. Die Eigenklebrigkeit kommt zustande, weil die Feuchtigkeit der Schleimhaut von den Polymeren der filmförmigen Darreichungsform aufgenommen wird. Dadurch beginnen diese zu quellen. Ferner umfasst die filmförmige Darreichungsform einen dehnbaren Polymerfilm, wobei der mindestens eine Wirkstoff in dem Polymer oder Polymergemisch gelöst oder fein verteilt vorliegt.

In einer Ausführungsform kann die filmförmige Darreichungsform mindestens ein in Wasser quellfähiges oder lösliches Polymer enthalten, das aus der Gruppe von Polymeren ausgewählt ist, die Dextran, Cellulosederivate, Polyacrylsäure, Polyacrylate, Polyethylenoxid-Polymere, Polyacrylamide, Polyethylenglykol, Kollagen, Alginate, Pectine, Traganth, Chitosan, Alginsäure, Arabinogalactan, Galactomannan, Agar-Agar, Agarose, Carrageen und natürliche Gummen umfasst. Die Cellulosederivate können aus der Gruppe ausgewählt sein, die Carboxymethylcellulose, Ethylcellulose und Propylcellulose umfasst.

In einer weiteren und/oder alternativen Ausführungsform kann die filmförmige Darreichungsform mindestens ein wasserlösliches oder zumindest teilweise wasserlösliches Polymer enthalten, das aus der Gruppe von Polymeren ausgewählt ist, die Polyvinylalkohol, Cellulosederivate, Stärke, Stärkederivate, Gelatine, Polyvinylpyrrolidone, Gummi arabicum, Pullulan und Acrylate umfasst. Die Cellulosederivate sind vorzugsweise aus der Gruppe ausgewählt, die Hydroxypropylcellulose, Hydroxypropylmethylcellulose, Natrium-Carboxymethylcellulose, Methylcellulose, Hydroxyethylcellulose und Hydroxypropylethylcellulose umfasst. Die Stärke oder Stärkederivate können aus der Gruppe ausgewählt sein, die Maltodextrine, Amylose, Amylopektin, Maisstärke, Kartoffelstärke, Reisstärke, Tapiokastärke, Erbsenstärke, Süßkartoffelstärke, Gerstenstärke, Weizenstärke, wachsige Maisstärke und modifizierte Stärke umfasst.

Die physikalischen Eigenschaften der filmförmigen Darreichungsform wie Mukoadhäsivität, Flexibilität, Löslichkeitsverhalten, Quellverhalten und dergleichen lassen sich durch die Kombination von zwei oder mehreren der vorgenannten Polymere entsprechend den Wünschen und Erfordernissen einstellen.

Die physikalischen Eigenschaften der filmförmigen Darreichungsform können auch durch den Gehalt an Polymer eingestellt werden. Der PolymerAnteil an der filmförmigen Darreichungsform beträgt mindestens 5 Gew.-%, vorzugsweise mindestens 10 Gew.-%, besonders bevorzugt mindestens 30 Gew.-%. Der Polymeranteil an der filmförmigen Darreichungsform beträgt nicht mehr als 90 Gew.-%, vorzugsweise nicht mehr als 70 Gew.-%, und besonders bevorzugt nicht mehr als 60 Gew.-%.

Die erfindungsgemäße Darreichungsform umfasst mindestens einen Wirkstoff, der zur Behandlung von Aphten geeignet ist. Der mindestens eine Wirkstoff liegt in dem Polymer beziehungsweise Polymergemisch in gelöster oder fein verteilter Form vor.

In einer weiteren und/oder alternativen Ausführungsform kann der mindestens eine Wirkstoff ein Gerbstoff sein. Unter einem Gerbstoff werden recht kompliziert aufgebaute chemische Substanzen verstanden, die in vielen Pflanzen vorkommen können. Gerbstoffhaltige Pflanzen und/oder Pflanzenteile, die zur Gewinnung von gerbstoffhaltigen Extrakten verwendet werden können, können beispielsweise aus der Gruppe ausgewählt sein, die Galläpfel, Hamamelisblätter, Walnussblätter, Eichenrinde, Rathaniawurzel, Blutwurzwurzel, Heidelbeeren, Brombeerblätter, Gänsefingerkraut, Erdbeerblätter, Odermennigkraut, Frauenmantelkraut, Breitwegereichblätter, Spitzwegereichblätter, Rosenblätter und Wiesenknopfblätter umfasst.

Gerbstoffe reagieren mit der oberen Schleimhautschicht und bilden eine Art Membran aus verfestigten Hauteiweißen (Gerbung). Ein Gerbstoff verbindet sich bei Kontakt mit Eiweiß mit diesem. Das in Proteinen gebundene Wasser kann durch Gerbstoffe verdrängt werden, so dass es zu einer Entwässerung kommt. Biologisch aktive Proteine können durch Gerbstoffe denaturiert werden, so dass sie nicht mehr biologisch aktiv sind.

In einer weiteren und/oder alternativen Ausführungsform kann der Gerbstoff ein Gerbstoff sein, der aus der Gruppe der Catechin-Gerbstoffe ausgewählt ist. In einer anderen und/oder zusätzlichen Ausführungsform kann der Gerbstoff Catechu sein. Der Gerbstoff Catechu wird aus der Gerberakazie, *Acacia catechu,* gewonnen. Dazu wird das Holz dieser tropischen Mimosenart ausgekocht und man erhält einen harzartigen Extrakt, der sehr gerbstoffhaltig ist.

In einer anderen und/oder zusätzlichen Ausführungsform kann der der mindestens eine Wirkstoff ein ätherisches Öl sein. Unter einem ätherischen Öl werden flüchtige, lipophile Pflanzeninhaltsstoffe mit angenehmem Eigengeruch verstanden. Ätherische Öle weisen antimikrobielle Aktivität auf. In einer Ausführungsform kann das ätherische Öl aus der Gruppe der ätherischen Öle ausgewählt sein, die in Kamillentinktur, Salbeitinktur, Melissentinktur, oder Arnikatinktur enthalten sind.

In einer weiteren und/oder alternativen Ausführungsform kann die filmförmige Darreichungsform einen Gehalt an Wirkstoff von mindestens 0,1 Gew.-%, vorzugsweise von mindestens 0,5 Gew.-% aufweisen. Die filmförmige Darreichungsform weist einen Gehalt an Wirkstoff von nicht mehr als 50 Gew.-% auf, vorzugsweise einen Gehalt an Wirkstoff von nicht mehr als 20 Gew.-% auf.

Eine einzelne Darreichungsform enthält vorzugsweise mindestens 0,5 mg Wirkstoff, vorzugsweise mindestens 1 mg Wirkstoff. Eine einzelne Darreichungsform enthält nicht mehr als 20 mg Wirkstoff, vorzugsweise nicht mehr als 10 mg Wirkstoff.

In einer weiteren und/oder alternativen Ausführungsform kann die filmförmige Darreichungsform einen oder mehrere pharmazeutisch annehmbare Hilfsstoffe umfassen. Die Art und der Gehalt an Hilfsstoffen hängen ebenso von dem Polymer oder Polymergemisch ab, welches die Darreichungsform aufweist, wie von dem mindestens einen Wirkstoff.

Der mindestens eine pharmazeutisch annehmbare Hilfsstoff kann aus der Gruppe ausgewählt sein, die Weichmacher, Stabilisatoren, Trägerstoffe, Geschmacksstoffe und Füllstoffe umfasst.

In einer weiteren und/oder alternativen Ausführungsform kann die filmförmige Darreichungsform mindestens einen Weichmacher als Hilfsstoff aufweisen. Der mindestens eine Weichmacher kann aus der Gruppe von Weichmachern ausgewählt sein, die Glycerin, höhere Alkohole, beispielsweise Dodecanol, Ester von Carbonsäuren, insbesondere Ester von Carbonsäuren, bei denen die Alkoholkomponente ein polyethoxylierter Alkohol ist, Ester von Dicarbonsäuren und Triglyceride, insbesondere mittelkettige Triglyceride der Capryl- und/oder Caprinsäure des Kokosöls, Propan-1,2-diol. Andere Polyethylenglykole sind in den Pharmakopöen erwähnt. Unter dem Begriff "höhere Alkohole" werden Alkohole verstanden, die bei Raumtemperatur, also bei einer Temperatur von 18°C bis 22°C, fest sind. Bei den höheren Alkoholen im Sinne der vorliegenden Offenbarung handelt es sich vorzugsweise um physiologisch unbedenkliche Alkohole. Der Begriff "mittelkettig" im Zusammenhang mit Triglyceriden bezeichnet die Länge der Fettsäurereste. Dabei haben mittelkettige Triglyceride Fettsäurereste mittlerer Länge, das bedeutet Fettsäurereste auf Basis von Fettsäuren mit 6 bis 12 Kohlenstoffatomen.

Der Gehalt an mindestens eine pharmazeutisch annehmbare Hilfsstoff in der filmförmigen Darreichungsform kann ein einer anderen und/oder alternativen Ausführungsform mehr als 1 Gew.-% und bis zu 10 Gew.-%, bis zu 20 Gew.-% oder bis zu 30 Gew.-% betragen.

In einer weiteren und/oder alternativen Ausführungsform kann die folienförmige Darreichungsform eine einheitliche Dicke aufweisen, die in einem Bereich von 10 µm bis 500 µm liegt, vorzugsweise in einem Bereich von 20 µm bis 300 µm.

Die einzelnen Darreichungsformen besitzen eine Fläche im Bereich zwischen 1 cm² und 10 cm², vorzugsweise im Bereich zwischen 5 cm² und 8 cm²_{.}

In einer weiteren und/oder alternativen Ausführungsform kann das topische Arzneimittel eine wiederablösbare Schutzschicht umfassen, auf der die folienförmige Darreichungsform ruht. Bei der wiederablösbaren Schutzschicht kann es sich um silikonisiertes Papier handeln.

In einer weiteren und/oder alternativen Ausführungsform kann die folienförmige Darreichungsform direkt auf der Innenseite der Verpackung ruhen, also auf der Fläche eines Packmittels, die einen Bereich des Innenraums der Verpackung, mit dem die folienförmige Darreichungsform verpackt ist, für die filmförmige Darreichungsform bereitstellt.

In einer weiteren und/oder alternativen Ausführungsform kann die ablösbare Schutzschicht, mit der die filmförmige Darreichungsform in Kontakt steht, aus denselben Materialien bestehen, die zur Herstellung von Prozesslinern genutzt werden, sofern diese ablösbar gemacht wurden, beispielsweise durch Silikonisierung. Ein Prozessliner ist eine Folie, die im Herstellungsverfahren von flächigen Arzneiformen zwar eingesetzt wird, im Endprodukt aber nicht enthalten ist, weil sie prozessbedingt wieder entfernt wurde. Bei der ablösbaren Schutzschicht kann es sich um eine Lage aus Polytetrafluorethylen, behandeltem Papier, Cellophan, Polyvinylchlorid oder ähnlichem handeln.

In einer weiteren und/oder alternativen Ausführungsform weist die ablösbare Schutzschicht eine Fläche auf, die größer ist als die Fläche der filmförmigen Darreichungsform in therapiegerechter Größe und in therapiegerechtem Format. Vorzugsweise ist die größere Fläche der ablösbaren Schutzschicht derart ausgestaltet, dass sie im Wesentlichen an einem Ende über die Fläche der filmförmigen Darreichungsform übersteht. Dieser überstehende Bereich der ablösbaren Schutzschicht ermöglicht ein leichteres abziehen der Schutzschicht von der filmförmigen Darreichungsform, insbesondere bei der Applikation der filmförmigen Darreichungsform.

In einer weiteren und/oder alternativen Ausführungsform ist die Darreichungsform einzeldosiert in einem Siegelrandbeutel verpackt. Gemäß einer anderen Ausführungsform liegen mehrere Darreichungsformen in einer gemeinsamen Verpackung vor.

Als Packstoff für die filmförmigen Darreichungsformen werden vorzugsweise siegelfähige Verbundpackstoffe verwendet. Bevorzugte Siegelmedien, die die siegelfähigen Verbundpackstoffe auf derjenigen ihrer Seiten aufweisen, die den Innenraum der späteren Verpackung ausbildet, sind Polyacrylnitril und Polyethylenterephthalat, weil diese während der Lagerung der verpackten filmförmigen Darreichungsformen kein oder nur sehr wenig ätherisches Öl aufnehmen.

Gemäß dem zweiten Aspekt stellt die Erfindung ein Verfahren zur Herstellung der zuvor beschriebenen filmförmigen Darreichungsformen bereit.

In einer Ausführungsform kann die erfindungsgemäße filmförmige Darreichungsform hergestellt werden, indem der mindestens eine Wirkstoff homogen in einer Lösung oder Suspension des Polymers oder der Polymermischung in einem Lösung- oder Suspendiermittel verteilt wird.

In einer weiteren und/oder alternativen Ausführungsform können der mindestens eine Wirkstoff zusammen mit dem mindestens einen pharmazeutisch annehmbaren Hilfsstoff homogen in einer Lösung oder Suspension des Polymers oder der Polymermischung in einem Lösung- oder Suspendiermittel verteilt werden.

Die resultierende Mischung aus Polymer beziehungsweise Polymermischung, mindestens einem Wirkstoff und optional mindestens einem pharmazeutisch annehmbaren Hilfsstoff kann auf ein Trägermaterial aufgestrichen werden. Daraufhin kann das Lösungs- oder Suspendiermittel aus der aufgestrichenen Mischung entfernt werden, so dass ein Polymerfilm erhalten wird, aus dem die filmförmigen Darreichungsformen vereinzelt werden können.

Gemäß dem dritten Aspekt umfasst die Erfindung die Verwendung der zuvor beschriebenen filmförmigen Darreichungsformen zur Behandlung von Aphten. Dazu kann eine der filmförmigen Darreichungsformen auf die zu behandelnde Aphte aufgebracht werden, so dass die Aphte von der filmförmigen Darreichungsform überdeckt wird. Aufgrund ihrer mukoadhäsiven Eigenschaften bleibt die filmförmige Darreichungsform in Kontakt mit der Schleimhaut und gibt den in ihr enthaltenen mindestens einen Wirkstoff an die betroffene Schleimhaut sowie an die Umgebung der Aphte ab. Die filmförmige Darreichungsform löst sich über einen längeren Zeitraum hinweg auf.

Gemäß dem vierten Aspekt erstreckt sich die Erfindung auch auf Verfahren zur Behandlung von Aphten, insbesondere zur Behandlung von Aphten im Mundraum, bei denen eine filmförmige Darreichungsform, bestehend aus einen Film aus einem quellfähigen oder wasserlöslichen Polymer oder Polymergemisch, und mindestens einen Wirkstoff, der aus der Gruppe ausgewählt ist, die aus Gerbstoffen und ätherischen Ölen besteht, auf die zu behandelnde Aphte aufgebracht wird, so dass die filmförmige Darreichungsform die Aphte überdeckt.

Die vorliegende Erfindung wird nachfolgend anhand von Ausführungsbeispielen veranschaulicht, wobei die Ausführungsbeispiele nur zur Erläuterung dienen, aber die vorliegende Erfindung nicht einschränken. Die vorliegende Erfindung wird ausschließlich durch die Ansprüche definiert.

### Beispiel 1

40 g Catechu (gepulvert), 10 g Zimtpulver und 150 g Polyvinylpyrrolidon K 90 wurden in 300 ml 45 %-igem Ethanol unter Rühren gelöst. Nach vollständigem Auflösen der Feststoffe entstand eine viskose Flüssigkeit, die mit einer Rakelwalze auf silikonisiertes Papier aufgetragen wurde. Durch mildes Trocknen wurde der größte Teil des Wassers und des Ethanols aus der viskosen Flüssigkeit entfernt, so dass ein Film enthalten wurde. Mit einem Locheisen wurden anschließend kreisrunde Gebilde mit einer Fläche von etwa 1 cm² ausgestanzt und mit ihrer Filmseite auf das Siegelmedium eines Verbundpackstoffs gelegt. Anschließend wurde das silikonisierte Papier entfernt und eine weitere Lage des Verbundpackstoffs auf den kreisrunden Film gelegt, so dass die Siegelflächen der beiden Verbundpackstofflagen einander zugewandt waren. Mit einer Siegelmaske wurden die Verbundpackstofflagen zu einem Siegelrandbeutel verschweißt, der den wirkstoffhaltigen Film enthielt.

### Beispiel 2

Es wurde eine filmförmige Darreichungsform gemäß Beispiel 1 hergestellt, wobei der Film folgende Zusammensetzung aufwies:
1,0 mg Catechu-Tinktur
6,5 mg Carbopol
2,5 mg Stärke.

## Patentansprüche

1. Topisches Arzneimittel zur Behandlung von Aphten, umfassend eine filmförmige Darreichungsform, die einen Film aus einem in Wasser quellfähigen oder wasserlöslichen Polymer oder einem in Wasser quellfähigen oder wasserlöslichen Polymergemisch, sowie mindestens einen Wirkstoff zur Behandlung von Aphten umfasst.

2. Topisches Arzneimittel gemäß Anspruch 1, **dadurch gekennzeichnet, dass** die filmförmige Darreichungsform mindestens ein in Wasser quellfähiges oder lösliches Polymer enthält, das aus der Gruppe ausgewählt ist, die Dextran, Cellulosederivate, Polyacrylsäure, Polyacrylate, Polyethylenoxid-Polymere, Polyacrylamide, Polyethylenglykol, Kollagen, Alginate, Pectine, Traganth, Chitosan, Alginsäure, Arabinogalactan, Galactomannan, Agar-Agar, Agarose, Carrageen und natürliche Gummen umfasst.

3. Topisches Arzneimittel gemäß Anspruch 1 oder 2, **dadurch gekennzeichnet, dass** die filmförmige Darreichungsform mindestens ein wasserlösliches oder zumindest teilweise wasserlösliches Polymer enthält, das aus der Gruppe ausgewählt ist, die Polyvinylalkohol, Cellulosederivate, Stärke, Stärkederivate, Gelatine, Polyvinylpyrrolidone, Gummi arabicum, Pullulan und Acrylate umfasst.

4. Topisches Arzneimittel gemäß einem der vorhergehenden Ansprüche, **dadurch gekennzeichnet, dass** der Gehalt an Polymer an der filmförmigen Darreichungsform mindestens 5 Gew.-%, vorzugsweise mindestens 10 Gew.-%, besonders bevorzugt mindestens 30 Gew.-% beträgt, und nicht mehr als 90 Gew.-%, vorzugsweise nicht mehr als 70 Gew.-%, und besonders bevorzugt nicht mehr als 60 Gew.-%.

5. Topisches Arzneimittel gemäß einem der vorhergehenden Ansprüche, **dadurch gekennzeichnet, dass** der mindestens eine Wirkstoff aus der Gruppe ausgewählt ist, die aus Gerbstoffen und ätherischen Ölen besteht.

6. Topisches Arzneimittel gemäß Anspruch 6, **dadurch gekennzeichnet, dass** der Gerbstoff aus der Gruppe der Catechin-Gerbstoffe ausgewählt ist.

7. Topisches Arzneimittel gemäß Anspruch 5 oder 6, **dadurch gekennzeichnet, dass** der Gerbstoff Catechu ist.

8. Topisches Arzneimittel gemäß Anspruch 6, **dadurch gekennzeichnet, dass** das ätherische Öl aus der Gruppe von ätherischen Ölen ausgewählt, die in Kamillentinktur, Salbeitinktur, Melissentinktur, oder Arnikatinktur enthalten sind.

9. Topisches Arzneimittel gemäß einem der vorhergehenden Ansprüche, **dadurch gekennzeichnet, dass** die filmförmige Darreichungsform einen Gehalt an Wirkstoff von mindestens 0,1 Gew.-%, vorzugsweise von mindestens 0,5 Gew.-%, und von nicht mehr als 50 Gew.-%, vorzugsweise von nicht mehr als 20 Gew.-% aufweist.

10. Topisches Arzneimittel gemäß einem der vorhergehenden Ansprüche, **dadurch gekennzeichnet, dass** die folienförmige Darreichungsform mindestens einen pharmazeutisch annehmbaren Hilfsstoff enthält, der vorzugsweise aus der Gruppe ausgewählt ist, die Weichmacher, Stabilisatoren, Trägerstoffe, Geschmacksstoffe und Füllstoffe umfasst.

11. Topisches Arzneimittel gemäß Anspruch 10, **dadurch gekennzeichnet, dass** der Weichmacher der Gruppe von Weichmachern ausgewählt ist, die Glycerin, bei Raumtemperatur feste Alkohole wie Dodecanol, Ester von Carbonsäuren, insbesondere Ester von Carbonsäuren, bei denen die Alkoholkomponente ein polyethoxylierter Alkohol ist, Ester von Dicarbonsäsuren und Triglyceride, insbesondere mittelkettige Triglyceride der Carbryl- und/oder Caprinsäure des Kokosöls, Propan-1,2-diol und andere Polyethylenglykole umfasst.

12. Topisches Arzneimittel gemäß Anspruch 10 oder 11, **dadurch gekennzeichnet, dass** der Gehalt an dem mindestens einen pharmazeutisch annehmbaren Hilfsstoff mehr als 1 Gew.-% und bis zu 30 Gew.-% beträgt.

13. Topisches Arzneimittel gemäß einem der vorhergehenden Ansprüche, **dadurch gekennzeichnet, dass** es eine ablösbare Schutzschicht umfasst, auf der die filmförmige Darreichungsform ruht.

14. Verfahren zur Herstellung eines topischen Arzneimittels gemäß einem der Ansprüche 1 bis 12, **dadurch gekennzeichnet, dass** in dem Verfahren der mindestens eine Wirkstoff und optional der mindestens eine Hilfsstoff homogen in einer Lösung oder Suspension des Polymers oder der Polymergemisch in einem Lösungs- oder Suspendiermittel verteilt wird, die resultierende Mischung auf ein Trägermaterial aufgestrichen, anschließend das Lösungs- oder Suspendiermittel entfernt wird, so dass ein Polymerfilm erhalten wird, und die Darreichungsformen daraus vereinzelt werden.

15. Verwendung eines topischen Arzneimittels gemäß einem der vorhergehenden Ansprüche zur Behandlung von Aphten.
